# FASCICULE DE BREVET EUROPEEN

(11) **EP 1 395 167 B1**
(45) Date de publication et mention de la délivrance du brevet: **12.09.2007**
(21) Numéro de dépôt: 02740816.0
(22) Date de dépôt: 27.05.2002
(51) Int. Cl.: A61B 1/005

(54) **STRUCTURE TUBULAIRE ACTIVE ORIENTABLE ET ENDOSCOPE FORME A PARTIR D'UNE TELLE STRUCTURE**
AKTIVE SCHWENKBARE ROHRSTRUKTUR UND DARAUS GEBILDETES ENDOSKOP
ACTIVE STEERABLE TUBULAR STRUCTURE AND ENDOSCOPE MADE FROM SAID STRUCTURE

(30) Priorité: 28.05.2001 FR 0106948
(43) Date de publication de la demande: 10.03.2004
(73) Titulaire: UNIVERSITE PIERRE ET MARIE CURIE, 75005 Paris (FR)
(72) Inventeur: BIDAUD, Philippe, F-94000 Creteil (FR); SZEWCZYK,Jérôme, F-95510 Vienne-en-Arthie (FR); BERTRAND, Jean-Claude, F-91470 Forges Les Bains (FR); TROISFONTAINE, Nelly, F-93360 Neuilly-Plaisance (FR); DE SARS, Vincent, 92120 Montrouge (FR)
(74) Mandataire: Abello, Michel
(86) Numéro de dépôt international: PCT/FR2002/001769
(87) Numéro de publication internationale: WO 2002/096276

(56) Documents cités:
- DE-A- 4 240 435
- US-A- 5 220 911
- US-A- 5 679 216
- US-A- 5 996 346

## Description

La présente invention concerne une structure tubulaire active orientable, et un endoscope formé à partir d'une telle structure.

Les endoscopes ou fibroscopes sont des instruments tubulaires destinés à explorer diverses canalisations, dans le cas d'endoscopes industriels, ou diverses cavités, canaux ou vaisseaux du corps humain dans le cas d'endoscopes médicaux. La tête des endoscopes est munie d'un dispositif lumineux, d'une caméra vidéo et éventuellement de micro-outils de chirurgie mini-invasive, tels que des micro-pinces, destinés à faire des prélèvements de tissus, des incisions ou à recoudre des tissus. Les canaux vidéo et les canaux de transmission de la lumière, ainsi que les différents câbles raccordés aux outils, sont disposés dans le corps de l'endoscope, qui peut être rigide ou souple, et sont connectés au niveau de l'extrémité proximale de l'endoscope à des systèmes de contrôle et de commande. La tête de l'endoscope est généralement articulée par l'intermédiaire d'une ou plusieurs paires de câbles commandées par l'extrémité proximale de l'endoscope.

Pour conférer une courbure voulue dans l'espace aux endoscopes ou fibroscopes orientables, il est connu de disposer des actionneurs en alliage à mémoire de forme le long de la structure tubulaire pour former des endoscopes dits actifs. L'utilisation de tels alliages à mémoire de forme, ci-après appelés AMF, est basée sur leur comportement thermomécanique, plus précisément leur capacité à subir des transformations microstructurelles en fonction de la température et de la contrainte mécanique qui leur sont appliquées, entre une phase austénite à haute température et une phase martensite à basse température. Ces transformations microstructurelles conduisent à une modification de la forme mais aussi au changement de plusieurs propriétés physiques de l'alliage, telles que la résistance électrique, le module d'Young ... Ce phénomène est dû à une réorganisation cristallographique sans modification de la composition chimique de l'alliage, suite à un déplacement collectif et coopératif des atomes sur une distance très faible par rapport aux paramètres de la maille cristalline. Lorsque l'AMF est chauffé au-dessus d'une température dite d'activation, et si l'AMF n'est pas excessivement déformé ou surcontraint, il se transforme d'un état souple, pliable correspondant à sa phase martensite, à un état dit mémorisé ou préprogrammé, plus rigide correspondant à sa phase austénite. Un état mémorisé donné de l'AMF est obtenu en le soumettant à une contrainte physique lors d'un traitement thermique à une température de recuit appropriée. Lorsque l'AMF refroidit, il passe en phase martensite et retrouve ses propriétés d'origine. Plus précisément, il passe à une phase martensite maclée après refroidissement en dessous d'une température dite de désactivation, sans perdre ses propriétés et c'est seulement après une contrainte extérieure qu'il retrouve ses propriétés d'origine. Le comportement thermodynamique de ces matériaux est fortement non linéaire à cause de la présence d'hystérésis importantes, et variable avec le nombre de cycles effectués.

Les différents types d'endoscopes ou fibroscopes actifs à ce jour sont basés sur l'utilisation d'actionneurs sous forme de fils en AMF disposés longitudinalement dont on fait varier la longueur en fonction de la température pour conférer au corps de l'endoscope la courbure voulue. Dans l'article "Micro Active Catheter System with Multi Degrees of Freedom", publié dans IEEE Int. Conf. On Robotic and Automation, vol 3, 1994, p 2290-2295, il est décrit un cathéter actif formé d'un tube flexible comprenant trois fils en AMF disposés dans des lumières du tube, parallèlement, à 120° les uns des autres selon l'axe du tube. Chaque fil peut être contracté par chauffage électrique pour engendrer une courbure au tube flexible.

Le brevet US 5 679 216 décrit un endoscope comprenant une pluralité de parties flexibles, par exemple formées par un ensemble de petits tubes cylindriques alignés, montés pivotant les uns par rapport aux autres, et disposés dans un tube flexible. Une pluralité de paires d'actionneurs formés par des fils linéaires en AMF, éventuellement sous forme hélicoïdale, sont disposées à l'intérieur des parties flexibles. Les fils en AMF de chaque paire sont disposés à 180° l'un de l'autre et sont fixés par leurs extrémités à deux parties flexibles adjacentes. Le chauffage sélectif d'actionneurs permet alors de conférer la courbure voulue à l'endoscope.

Selon un autre type d'endoscope ou fibroscope, décrit notamment dans le brevet US 5 405 337, un tube flexible est recouvert d'un film flexible sur lequel sont répartis différents fils en AMF.

De tels fils en AMF présentent des allongements en fonction de la température qui sont relativement restreints, de l'ordre de 4 à 5 % de la longueur des fils. Pour obtenir des actionneurs produisant des déplacements et des efforts suffisants, il est alors nécessaire d'avoir des fils relativement longs. Dans le cas d'un endoscope formé d'une pluralité de parties flexibles adjacentes, tel que décrit dans le brevet US 5 679 216 précité, la longueur des fils impose la longueur des parties flexibles, or on souhaite limiter la longueur desdites parties flexibles pour pouvoir en empiler davantage dans le but d'avoir plus de degré de liberté. Par ailleurs, le diamètre externe de l'endoscope doit être aussi petit que possible pour permettre par exemple des applications en neurochirurgie ou en chirurgie cardio-vasculaire, tout en maintenant un espace interne suffisant pour le passage des différents câbles optiques et des canaux pour la commande des micro-outils. La nature des actionneurs et leur disposition dans les endoscopes décrits précédemment ne permettent pas d'avoir une structure compacte. De tels actionneurs limitent inévitablement l'espace interne de l'endoscope ou augmentent le diamètre externe de celui-ci.

Le but de la présente invention est de proposer une structure tubulaire active palliant aux inconvénients de l'art antérieur, disposant d'un grand nombre de degrés de liberté afin de contourner les obstacles et d'adopter des configurations qui limitent les interactions avec l'environnement.

Ce but est atteint par l'invention telle que définie dans la revendication 1.

Selon une particularité, entre chaque paire d'unités modulaires, sont montés deux moyens de rappel de part et d'autre de leur axe de rotation commun, les positions d'alignement et d'orientation desdites unités modulaires correspondant à la position d'équilibre des deux moyens de rappel.

Avantageusement, chaque unité modulaire présente une forme générale annulaire creuse, de sorte que leur empilement définisse un volume interne utile.

Selon un mode de réalisation, chaque moyen de rappel est constitué par au moins un ressort, assemblé par ses extrémités longitudinales à deux maillons consécutifs, sollicité de préférence en traction à basse température, le pivotement relatif de deux maillons étant obtenu par augmentation de la raideur d'un des deux ressorts à effets antagonistes par chauffage de celui-ci au-delà de la température d'activation, la course retour en sens inverse étant obtenue après refroidissement du ressort activé en dessous d'une température de désactivation et par le rappel élastique de l'autre ressort antagoniste non activé.

Selon un mode de réalisation, chaque maillon comprend deux languettes mâles diamétralement opposées et disposées longitudinalement et deux encoches femelles de forme sensiblement complémentaire diamétralement opposées, disposées sensiblement à 90° desdites languettes mâles, orientées en sens inverse de celles-ci et dans lesquelles sont emboîtées les languettes mâles du maillon adjacent pour former une liaison pivot entre les deux maillons définissant leur axe de rotation.

Selon une particularité, chaque languette mâle possède une portion périphérique circulaire s'étendant sur un arc de cercle supérieur à 180°, de sorte que l'emboîtement desdites portions d'un maillon dans les encoches femelles complémentaires du maillon adjacent permet de retenir longitudinalement les deux maillons.

Avantageusement, chaque maillon comporte une bague comprenant deux parties diamétralement opposées venant transversalement en vis-à-vis des encoches femelles dudit maillon afin d'empêcher un déboîtement transversal des languettes mâles du maillon adjacent.

Selon une autre particularité, les extrémités longitudinales des ressorts sont assemblées aux maillons par l'intermédiaire de plots en céramique, lesdits plots portant des fils et connexions électriques, constituant les moyens de chauffage, qui permettent le chauffage desdits ressorts.

Avantageusement, chaque moyen de rappel comprend deux ressorts, disposés symétriquement par rapport à un plan perpendiculaire à l'axe de rotation commun des deux maillons auxquels ils sont associés, et assemblés auxdits maillons par les mêmes plots en céramique.

Selon une autre particularité, chaque ressort présente une forme générale repliée en accordéon et est constitué d'un ensemble de bandes flexibles superposées, reliées par des parties de liaison rigides.

Selon une autre particularité, la structure tubulaire active comprend au niveau des actionneurs des capteurs d'orientation destinés à être reliés aux moyens de commande pour le contrôle de l'orientation relative des unités modulaires, tels que des capteurs de flexion permettant de déterminer la position angulaire relative entre deux unités modulaires consécutives et/ou des capteurs de contact permettant de mesurer localement la force d'interaction entre la surface extérieure de la structure tubulaire et le milieu extérieur, et/ou des capteurs de température sur chaque actionneur.

Avantageusement, la structure tubulaire active comprend un bus de communication par multiplexage disposé dans le volume interne et destiné à assurer la liaison entre les moyens de commande externes, les moyens de chauffage et les capteurs pour commander électriquement, de manière sélective, l'activation des actionneurs en fonction des informations fournies par les capteurs et des ordres de commande fournis par l'utilisateur.

L'invention propose, par ailleurs, un endoscope comprenant un corps tubulaire longitudinal muni à son extrémité distale d'une tête dans laquelle est disposé un système de vision destiné à être relié par des canaux de communication à une unité de contrôle et de commande externe et/ou un ou plusieurs micro-outils destinés à être reliés par des câbles électriques à ladite unité de commande et de contrôle externe, lesdits canaux et/ou câbles étant disposés dans le corps tubulaire, caractérisé par le fait qu'au moins une partie du corps de l'endoscope est formée à partir de la structure tubulaire active orientable selon l'une des revendications 1 à 12, les unités modulaires de la structure étant recouvertes par au moins une gaine externe flexible.

L'invention sera mieux comprise, et d'autres buts, détails, caractéristiques et avantages apparaîtront plus clairement au cours de la description explicative détaillée qui va suivre d'un mode de réalisation particulier actuellement préféré de l'invention, en référence au dessin schématique annexé.

Sur ce dessin :
- la figure 1 représente une vue en perspective de deux maillons d'une structure tubulaire selon l'invention, munis de bagues et assemblés l'un à l'autre, et de deux actionneurs formés chacun d'une paire de ressorts, avant assemblage entre les deux maillons ;
- la figure 2 représente une vue de côté du maillon inférieur de la figure 1 selon un plan de coupe II-II, illustrant le positionnement d'une paire de ressorts de l'actionneur ;
- la figure 3A représente une vue en perspective d'un maillon ;
- les figures 3B et 3C représentent des vues en coupe, respectivement selon les plans III-B et III-C de la figure 3A ;
- la figure 4A représente une vue en perspective d'une bague interne ;
- les figures 4B et 4C représentent des vues en coupe, respectivement selon les plans IV-B et IV-C de la figure 4A ;
- la figure 5 représente une vue de côté d'un ressort selon l'invention ;
- la figure 6 représente une vue partielle de dessus de la figure 2 illustrant le positionnement d'une paire de ressorts sur un plot en céramique ;
- la figure 7 est une vue schématique en perspective d'un endoscope formé à partir d'une structure tubulaire selon l'invention ;
- la figure 8 est une vue schématique en coupe transversale partielle de l'endoscope illustré à la figure 7 ;
- la figure 9 représente les courbes de contrainte-déformation en phase martensite et en phase austénite d'un matériau à mémoire de forme, utilisable pour réaliser les ressorts selon l'invention ; et,
- la figure 10 représente de manière schématique la courbe de variation de la raideur d'un ressort en matériau à mémoire de forme en fonction de la température.

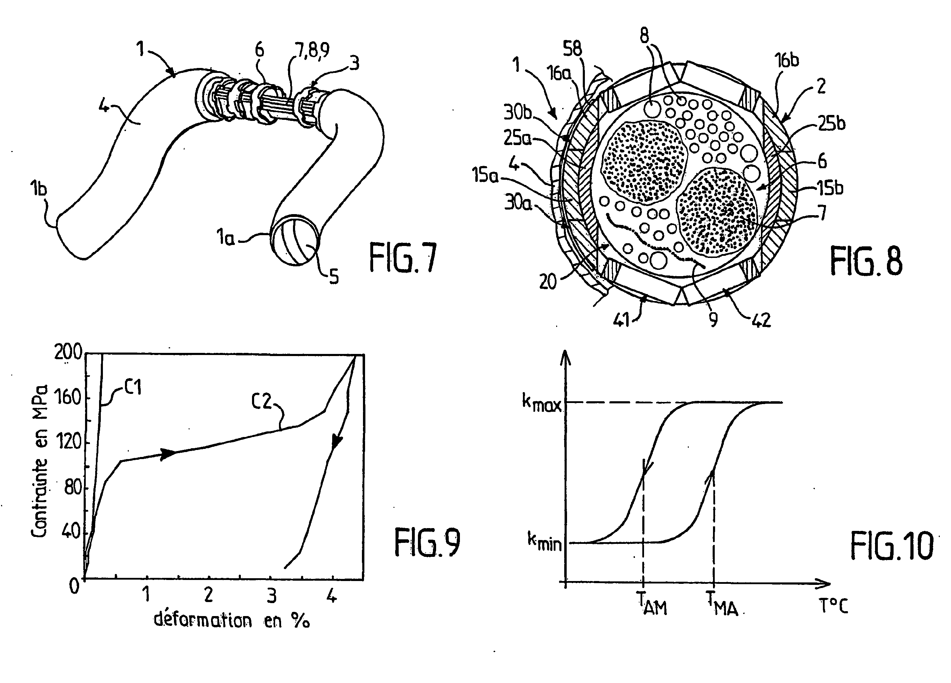

Les figures 7 et 8 représentent de manière schématique un endoscope médical 1 selon l'invention, formé à partir d'une structure tubulaire 2 longitudinale rigide comprenant un ensemble d'unités modulaires articulées entre elles, deux unités modulaires successives étant aptes à pivoter selon un axe de rotation commun. L'articulation entre deux unités modulaires successives peut être une articulation en deux pièces distinctes, chaque pièce constituant une unité modulaire, et deux pièces successives étant reliées entre elles par une liaison pivot ou une charnière sous forme de film, ou toute autre articulation venue de moulage. Dans le mode de réalisation illustré, les unités modulaires sont constituées de maillons ou anneaux rigides 3, sensiblement identiques, par exemple métalliques, reliés les uns aux autres par une liaison pivot, tel que décrit ci-après. Les maillons 3 sont recouverts d'une gaine externe 4, isolante et flexible, de nature élastomère. Dans le cas d'un endoscope industriel, appelé également boroscope, cette gaine externe peut être de nature métallique. L'extrémité distale la de la structure tubulaire est munie d'une tête 5 dans laquelle est placé un système de vision, comprenant une caméra et un système d'éclairage, et éventuellement un ou plusieurs micro-outils. Le volume interne 6 défini par les maillons est réservé à différents canaux de communication comprenant des paquets de fibres optiques 7 pour le système de vision et différentes liaisons électriques 8 pour la commande des micro-outils, ainsi qu'un bus de communication 9 pour la commande d'actionneurs distribués le long de la structure tubulaire tel que décrit ci-après. Le bus de communication 9, ainsi que les paquets de fibres optiques 7 et les liaisons électriques 8, sont connectés au niveau de l'extrémité proximale 1b de l'endoscope à une unité de commande et de contrôle externe (non représentée).

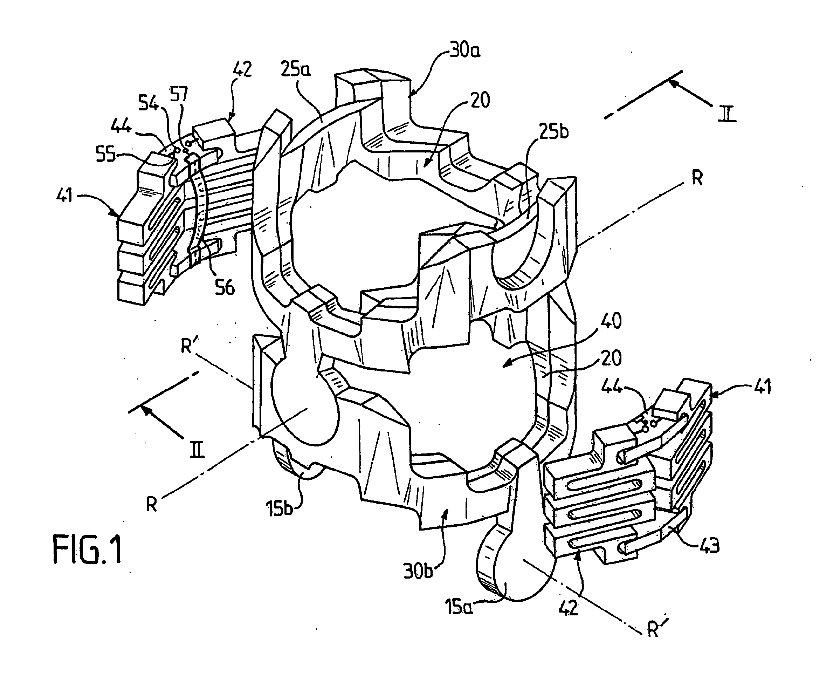

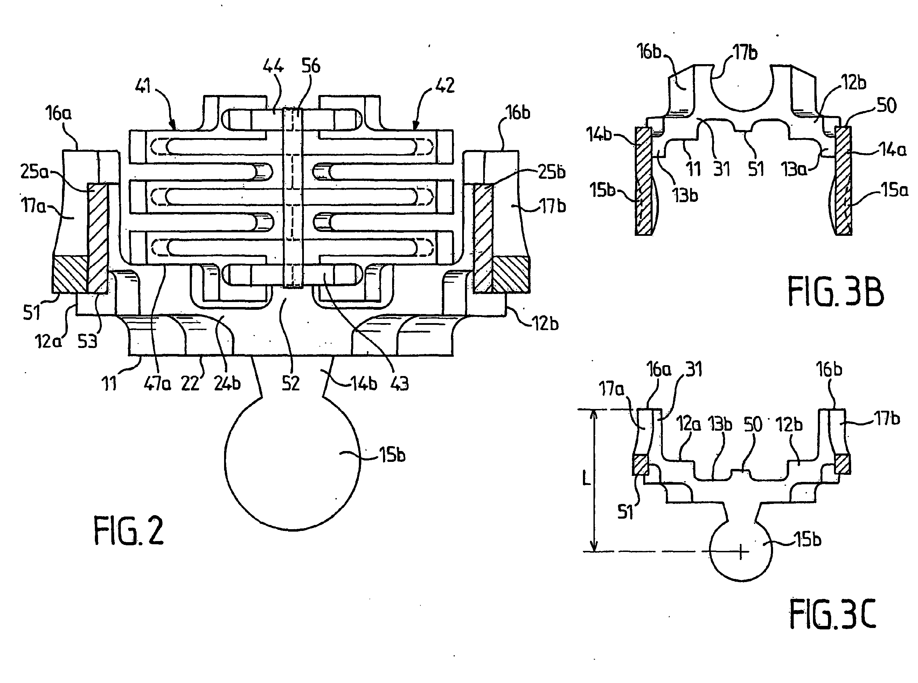

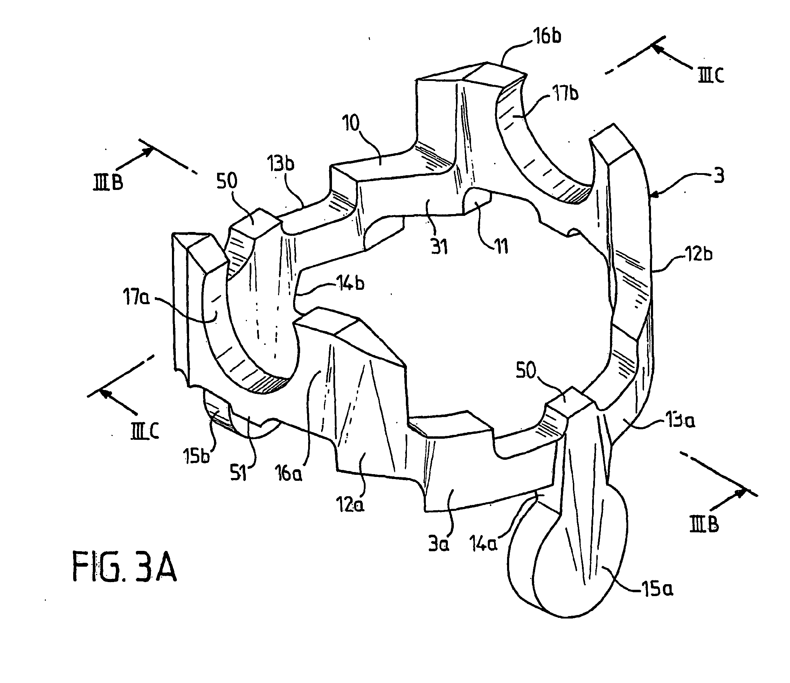

En référence aux figures 3A-C, chaque maillon 3 comprend une paroi 3a, de forme générale circulaire, comprenant un bord supérieur 10 et un bord inférieur 11. La paroi 3a est recourbée dans un même sens longitudinal, symétriquement par rapport à un plan IIIB, le bord supérieur étant orienté selon ledit sens longitudinal. Le maillon 3 est par la suite défini comme comprenant deux parties recourbées 12a, 12b diamétralement opposées, reliées par deux ponts 13a, 13b. Les ponts 13a, 13b comportent au niveau du bord inférieur 11 deux languettes mâles longitudinales 14a, 14b, diamétralement opposées, lesdites languettes se terminant par une portion périphérique circulaire 15a, 15b plus large, s'étendant sur un arc de cercle de plus de 180°, de préférence de plus de 270°. Les parties recourbées 12a, 12b présentent au niveau du bord supérieur 10 deux projections 16a, 16b, diamétralement opposées, disposées sensiblement à 90° des languettes mâles 14a, 14b, ces projections comprenant des encoches femelles circulaires 17a, 17b aptes à recevoir les portions circulaires d'un autre maillon. Les encoches comportent un bord circulaire s'étendant sur un arc de cercle supérieur à 180 ° , et inférieur à celui des portions circulaires. Telle que représentée sur la figure 1, la liaison entre deux maillons 30a, 30b est obtenue en insérant les portions circulaires d'un maillon dit supérieur 30a dans les encoches du maillon dit inférieur 30b. Lorsque les portions circulaires du maillon supérieur 30a sont disposées dans les encoches du maillon 30b, les bords en vis-à-vis des deux maillons sont espacés d'une distance permettant une rotation relative des deux maillons l'un par rapport à l'autre selon un axe de rotation transversal (R) passant par les deux portions circulaires. La rotation relative maximale de deux maillons, par exemple de ± 15°, est limitée par des butées mécaniques constituées par la venue en butée de chaque languette mâle 14a, 14b du maillon supérieur 30a contre les arêtes formées par le bord supérieur du maillon inférieur 30b et le bord circulaire de ses encoches et/ou par la venue en contact du bord inférieur des ponts du maillon supérieur avec le bord supérieur des projections du maillon inférieur. De part cette disposition à 90° des encoches par rapport aux portions circulaires, la structure tubulaire selon l'invention, obtenue par plusieurs maillons reliés les uns aux autres, présente des axes de rotation transversaux R, R' alternés de 90°.

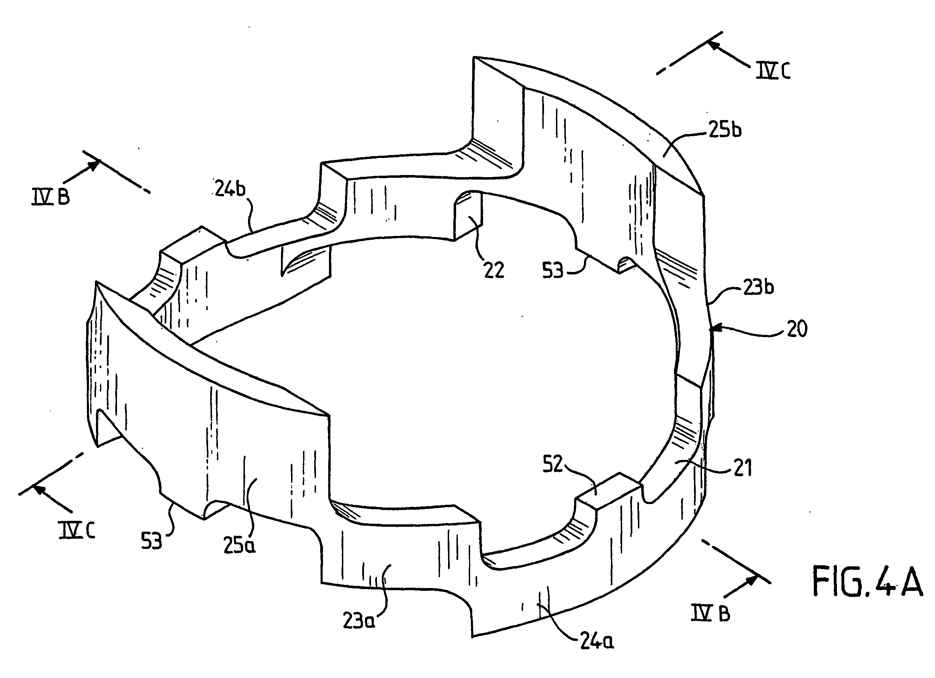

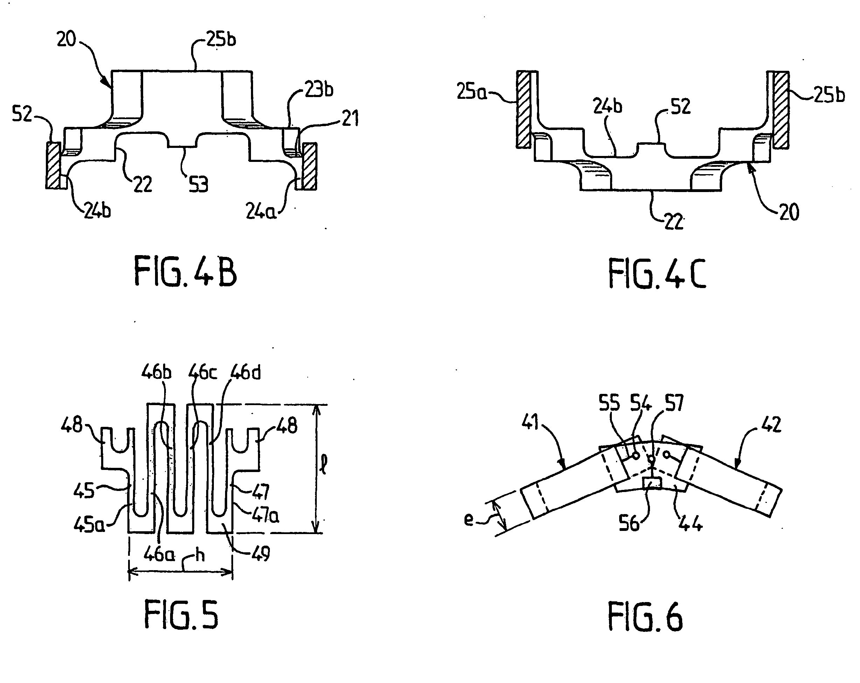

Pour assurer le positionnement transversal des deux maillons 30a, 30b selon leur axe de rotation R, une bague interne 20 est placée contre la face interne 31 des maillons, ladite bague interne comprenant deux parties diamétralement opposées venant en vis-à-vis de la face interne des portions circulaires. En référence aux figures 4A-C, la bague interne 20 présente une forme sensiblement similaire à celle d'un maillon, son diamètre externe étant sensiblement identique au diamètre interne d'un maillon. La bague 20 comprend une paroi 20a, comportant un bord supérieur 21 et un bord inférieur 22, recourbée dans un même sens longitudinal, symétriquement par rapport à un plan IVB, le bord supérieur étant orienté selon ledit sens longitudinal. Le bord supérieur 21 des parties recourbées 23a, 23b reliées par deux ponts 24a, 24b, possède deux projections 25a, 25b. Lorsque la bague est emboîtée dans un maillon, ces deux projections sont disposées contre la face interne des projections du maillon munies d'encoches, tel qu'illustré à la figure 1. L'assemblage de deux maillons s'effectue en emboîtant transversalement les extrémités circulaires du maillon supérieur 30a dans les encoches du maillon inférieur 30b et en insérant ensuite une bague interne dans le maillon inférieur pour empêcher un déboîtement transversal des deux maillons. La bague interne précitée peut être remplacée par une bague externe venant contre la face externe des maillons. Dans une variante de réalisation, chaque languette mâle comporte une saillie conique orientée vers l'extérieur. L'assemblage de deux maillons s'effectue alors sans utiliser de bague, en emboîtant longitudinalement les languettes mâles d'un maillon supérieur entre les projections munies d'encoches du maillon inférieur pour amener les saillies coniques dans les encoches. Lors de cette opération d'assemblage, les languettes mâles se déforment élastiquement jusqu'à ce que les saillies viennent s'insérer dans les encoches.

Les deux maillons, formés d'une paroi recourbée et ainsi assemblés, définissent deux espaces 40 à dimensions variables, diamétralement opposés, disposés de part et d'autre de l'axe de rotation R. Ces espaces s'étendent du bord inférieur des parties recourbées du maillon supérieur, au bord supérieur des ponts du maillon inférieur.

Selon l'invention, des actionneurs en alliage à mémoire de forme sont montés en antagoniste de part et d'autre de l'axe de rotation R de la liaison pivot entre deux maillons 30a, 30b. Ces actionneurs sont logés dans les espaces 40 précités entre les maillons. Chaque actionneur est formé d'une paire de ressorts 41, 42, dont les extrémités longitudinales sont assemblées au bord inférieur du maillon supérieur 30a et au bord supérieur du maillon inférieur 30b par l'intermédiaire de plots en céramique 43, 44. Les extrémités inférieures des ressorts d'une même paire sont assemblées à un même plot inférieur 43, et les extrémités supérieures des ressorts d'une même paire sont assemblées à un même plot supérieur 42. L'assemblage des actionneurs entre les maillons s'effectue par collage du plot supérieur 42 au bord inférieur du maillon supérieur 30a et par collage du plot inférieur 43 au bord supérieur du maillon inférieur 30b.

En référence aux figures 1 et 5, chaque ressort 42 est, de forme générale, plié en accordéon. Chaque ressort est formé de bandes flexibles sensiblement parallèles comprenant deux bandes d'extrémités 45, 47 et un ensemble de bandes intermédiaires 46a-d, reliées par des parties de liaison rigides 49, l'orientation de chaque partie de liaison restant constante lorsque le ressort est étiré pour des raisons de symétrie. Les bandes d'extrémités présentent une longueur inférieure à celle des bandes intermédiaires et sont chacune munies sur leur face externe 45a, 47a d'une patte rigide 48, de sorte que les plots 43, 44 puissent être enserrés entre l'extrémité libre d'une bande d'extrémité 45 et sa patte rigide 48 pour permettre l'assemblage des plots et des ressorts. Cet assemblage peut être complété par un collage. Suivant les figures 1 et 6, chaque plot 44 est formé d'une plaque correspondant sensiblement à un secteur de couronne. La largeur des plots est inférieure ou égale à l'épaisseur de la structure tubulaire, qui correspond à l'épaisseur des maillons 3 additionnée de celle des bagues internes 20. Les ressorts 41, 42 sont disposés symétriquement sur les plots 43, 44, selon une disposition qui correspond au rayon de courbure de la structure tubulaire, de telle sorte que la paire de ressorts soit sensiblement intégrée à la structure tubulaire sans limiter le volume interne 6 utile. Les deux ressorts 42 de la paire peuvent éventuellement venir en contact par leurs parties de liaison 49 disposées en vis à vis. Les ressorts peuvent éventuellement comprendre une partie légèrement saillante par rapport à la surface externe des maillons, ladite partie s'enfonçant dans la gaine externe 4, tel qu'illustré à la figure 8.

Les bords supérieurs 10, 21 des ponts 13a, 13b, 24a, 24b du maillon 3 et de la bague 20 possèdent une saillie 50, 52 sur laquelle le plot inférieur 43 est collé. De même, les bords inférieurs 11, 22 des parties recourbées 16a, 16b, 23a, 23b du maillon 3 et de la bague 20 présentent également une saillie 61, 53 pour le collage du plot supérieur 44. Le bord supérieur des ponts du maillon et de la bague, ainsi que le bord inférieur des parties recourbées du maillon et de la bague sont configurés de telle sorte que les faces externes 45a des bandes d'extrémités 45 des ressorts viennent en contact avec celles-ci. Ce positionnement d'un actionneur sur le maillon inférieur 30b est illustré à la figure 2. Les dimensions d'un actionneur par rapport à celles des espaces 40 à dimensions variables sont déterminées de sorte que les ressorts soient plus petits que les espaces 40 lorsque les deux maillons sont dans une position dite d'alignement dans laquelle leurs axes de symétrie sont confondus. Dans cette position d'alignement, à basse température, les ressorts fixés par l'intermédiaire des plots sont donc étirés, sous contrainte.

Les plots de céramique 44, 45 supportent les connections . électriques permettant le chauffage électrique par effet joule des ressorts 41, 42. Le plot inférieur 44 supporte un fil (non visible) reliant électriquement les extrémités inférieures des ressorts d'une paire, par exemple par leur patte rigide 48. Le plot supérieur 44 comprend deux bornes de connexion 54, chacune reliée par un fil 55 à une extrémité supérieure d'un ressort de la paire, par exemple les pattes rigides 48. Ces bornes sont destinées à permettre la connexion de fils électriques de chauffage (non représentés) reliés au bus de communication 9.

Suivant l'invention, le couple moteur permettant le pivotement d'un maillon par rapport à l'autre est produit par un déséquilibre des efforts de traction exercés par les deux paires de ressorts à effets antagonistes. Ce déséquilibre est obtenu en augmentant la rigidité des deux ressorts d'une des deux paires à effets antagonistes, en les alimentant électriquement par les bornes 54. L'apport d'énergie thermique par effet joule entraîne une transformation du matériau constituant les ressorts de sa phase martensite basse température souple vers sa phase austénite haute température, ce qui provoque une élévation du module d'Young du matériau et donc une augmentation de la rigidité des ressorts activés, dits à l'état actif. Les ressorts à l'état actif, à haute température, se contractent, générant ainsi un déplacement des éléments de structure auxquels ils sont solidarisés, à savoir un pivotement des deux maillons selon leur axe de rotation R. Lors de ce pivotement, les ressorts à effets antagonistes, à basse température, à l'état inactif, s'étirent davantage. Il est ainsi possible d'obtenir une position d'orientation relative souhaitée des deux maillons en faisant varier la raideur des ressorts d'une paire en fonction de la température. La course retour, correspondant au pivotement en sens inverse vers la position d'alignement initiale des deux maillons, est obtenue par refroidissement des ressorts à l'état actif en supprimant l'apport d'énergie et par l'effort de rappel élastique produit par la paire de ressorts à effets antagonistes à l'état inactif, ainsi que par la gaine externe 4.

Le chauffage sélectif d'un actionneur constitué d'une paire de ressorts permet de courber localement la structure tubulaire 2. Ainsi, le chauffage sélectif de plusieurs actionneurs répartis de long de la structure tubulaire permet de donner la forme tridimensionnelle voulue à la structure tubulaire.

Le temps nécessaire pour rigidifier un ressort dépend de la quantité d'énergie électrique fournie, tandis que le temps de retour à l'état inactif dépend du mode d'évacuation de la quantité d'énergie emmagasinée. L'énergie nécessaire au fonctionnement des ressorts est en partie évacuée par convection, ce qui a pour conséquence d'augmenter la température de l'endoscope. Cette température de l'endoscope doit rester entre deux températures seuils, à savoir entre la température du milieu, qui est celle du corps humain, et une température limite du point de vue sécurité, au-dessus de laquelle les organes et/ou tissus pourraient être abîmés. Le choix des températures de transition entre les deux phases, à savoir de la phase martensite vers la phase austénite (Tma) et de la phase austénite vers la phase martensite (Tam), qui conditionnent la quantité d'énergie évacuée et donc la température de l'endoscope, devra tenir compte de ces deux contraintes. Cependant, la température de transition (Tam) de la phase martensite vers la phase austénite sera de préférence choisie la plus élevée possible pour diminuer les temps de refroidissement des ressorts nécessaires au retour à l'état inactif desdits ressorts. Ces deux températures de transition peuvent être sensiblement égales, suivant le traitement thermique et/ou chimique appliqué au matériau.

La bande passante de ces actionneurs est définie par le temps de réponse en descente qui est fonction des échanges thermiques avec le milieu dans lequel l'actionneur est intégré. Pour des faibles quantités de matières et des surfaces de convection importantes comme pour les actionneurs formés par les paires de ressorts décrits précédemment, la bande passante, pour des amplitudes maximales de déplacement de deux maillons successifs de l'ordre de 15°, est de l'ordre du hertz. Cette bande passante peut être significativement améliorée en introduisant une boucle interne en température qui limite l'énergie électrique fournie à l'actionneur en évitant de parcourir des cycles d'hystérésis internes.

Le comportement non linéaire de ces actionneurs en AMF peut être maîtrisé par des régulateurs simples connus, compatible avec les contraintes d'intégration, pour obtenir une commande en position et en effort avec une bonne précision et stabilité. De tels régulateurs sont par exemple décrits par N.Troisfontaine, dans "Conception et contrôle de micro-actionneurs à alliage à mémoire de forme (AMF)", thèse présentée à l'Université Pierre et Marie Curie, Paris, Décembre 97.

Les dimensions et la disposition des ressorts sont telles que les actionneurs sont intégrés à la structure mécanique formée par les maillons, en préservant le volume interne utile 6, sensiblement sans en augmenter l'encombrement, et tout en garantissant un couple moteur satisfaisant, fonction de la nature et de l'épaisseur de la gaine externe, pour permettre la rotation de deux maillons.

A titre d'exemple illustratif, les maillons 3 et les bagues 20 internes peuvent être par exemple obtenus à partir de tube d'acier ou par moulage. Chaque maillon présente une longueur L (figure 3C), correspondant à la distance entre le bord supérieur des secondes projections et le centre des extrémités circulaires, comprise entre 4 et 5 mm, par exemple 4,5 mm. Un maillon muni d'une bague présente un diamètre interne de 5,4 mm et un diamètre externe de 7 mm. Le diamètre externe total de l'endoscope avec la gaine externe est de 8 mm.

Chaque ressort peut être obtenu par électroérosion à partir de bandes d'alliage Ni-Ti, préalablement recuites à haute température, selon des méthodes connues, afin de programmer la forme du matériau à haute température. Un tel procédé permet d'obtenir des découpes très précises. Le matériau utilisé pour la fabrication des ressorts est, par exemple, un alliage 50/50 de Nickel-Titane. Pour une contrainte nulle appliquée au matériau choisi, les températures de transition austénite vers martensite (Tam) et martensite vers austénite (Tma) sont respectivement de 45°C et 70°C. Les caractéristiques contrainte-déformation de cet alliage sont données sur la figure 9, en phase martensite et en phase austénite. Le Module de Young du ressort contraint en phase austénite à haute température correspond à la pente de la courbe à haute température (C1) tandis que le module de Young du ressort étiré en phase martensite correspond à la pente de la courbe à basse température (C2). Le Module d'Young du ressort varie de 20 à 70 Gpa en fonction de la température.

La contrainte maximale admissible retenue pour le dimensionnement des ressorts est de 135 MPa. Chaque ressort comprend 6 bandes, à savoir deux bandes d'extrémité 45, 47 et quatre bandes intermédiaires 46a-d, d'une épaisseur de 0,125 mm et espacées de 0,25 mm, soit une hauteur (h) de ressort de 2 mm. Le ressort présente une épaisseur (e) de 0,3 mm et une largeur (1) de 2,35 mm. Chaque ressort d'une paire délivre un couple de 0,008 Nm sur une plage angulaire de ± 15°. La figure 10 illustre schématiquement la variation de la raideur d'un ressort en fonction de la température.

Pour assurer le contrôle de l'orientation relative des maillons et ainsi le déplacement dans l'espace de la structure tubulaire selon l'invention, chaque actionneur entre deux maillons est asservi par trois types de capteur d'orientation qui s'intègrent dans la structure tubulaire sans gêner son déplacement. Ces capteurs sont reliés à l'unité de contrôle et de commande externe par l'intermédiaire du bus de communication 9.

Un premier capteur, dit de flexion, permet de connaître la position angulaire relative entre deux maillons. L'information retournée à l'unité de contrôle est de préférence de type résistif afin de simplifier l'électronique distribuée. Le capteur de flexion est, par exemple, constitué par une bande formée d'une lame de plastique souple sur laquelle est déposé un matériau résistif. Suivant la figure 1, la bande 56 est fixée en flexion par ses extrémités au plot supérieur 44 et au plot inférieur 43 d'au moins l'une des paires de ressorts 41, 42 disposées de part et d'autre de l'axe de rotation de deux maillons. Lorsque le matériau résistif voit son fléchissement varier, lors de l'étirement ou de la contraction de la paire de ressorts, sa résistance augmente. Les extrémités de la bande sont reliées au bus de communication 9 par l'intermédiaire de bornes de connexion 57 portées par les plots 43, 44. A titre d'exemple, le produit commercialisé par la société Images Compagny, sous la dénomination commerciale Sensorflex, peut être utilisé pour former le capteur de flexion. Ce produit se révèle facilement manipulable et garde ses propriétés quelle que soit la taille de l'échantillon, ce qui facilite son intégration dans la structure tubulaire. Par ailleurs, l'ordre de grandeur des variations de résistance d'un tel capteur facilite l'exploitation des mesures. Un tel capteur assure une résolution de l'ordre du degré sur la plage de variations d'environ ± 15° définie par les butées mécaniques précitées, qui est particulièrement adaptée pour le type d'environnement dans lequel l'endoscope va évoluer. Par ailleurs, ces capteurs distribués tout le long de la structure sont de dimensions réduites et facilement intégrables et ne gênent pas les déplacements de la structure tubulaire.

Dans une variante de réalisation, le capteur de flexion est un capteur piézoélectrique qui retourne une différence de potentiel sous un effort de flexion. Un tel capteur offre une grande précision, toutefois son intégration dans la structure est plus compliquée que celle du capteur précité de part la nature électrique et non résistive de la grandeur retournée, sa faible sensibilité de quelques mV, ainsi que sa difficulté de manipulation car ils ne peuvent être découpés.

Un second capteur, dit de contact, permet de mesurer localement la force d'interaction de l'endoscope avec l'environnement. Ces capteurs doivent êtres sensibles aux efforts perpendiculaires ainsi qu'aux efforts tangentiels dus aux frottements de l'endoscope sur une paroi, l'idéal étant de pouvoir mesurer en direction et en amplitude l'effort détecté par le capteur. La sensibilité des capteurs, de l'ordre du touché humain, doit être inférieure à l'effort minimal provoquant des lésions, cet effort étant de l'ordre d'un dixième de Newton. Les capteurs sont par exemple sous forme de films PZT sur des supports fins et souples, par exemple en polyimide, positionnés entre le maillon et la gaine extérieure, tel que représenté schématiquement par la référence 58 sur la figure 8. Ainsi, il est possible de commander des maillons en fonction des interactions avec l'environnement détectées par les capteurs de contact afin de courber localement la structure.

Dans une variante de réalisation, les capteurs de contact sont formés d'une bande de matériau résistif, telle que celle utilisée pour l'obtention des capteurs de flexion, ladite bande étant positionnée sur la surface de la gaine externe 4. Dans ce cas, les fils de connexion des capteurs traversent la gaine externe. Les capteurs sont positionnés sous une forme convexe. Lorsqu'un effort extérieur vient déformer la géométrie du capteur, l'écart de résistance relevé lors de la déformation est proportionnel à la force d'interaction. Dans une variante, la bande de matériau résistif est disposée à plat sur la surface de la gaine externe 4, en intercalant un matériau compliant entre la bande et la surface de la gaine externe. Une pression extérieure va déformer la bande que le matériau compliant va redresser. Entre temps, la déformation de la bande aura provoqué une variation de résistance représentative de la pression exercée.

Les capteurs de contact peuvent également être constitués de capteurs à encre sous la forme de dalle dont la résistance varie en fonction de l'intensité de la force appliquée.

La modification de la rigidité d'un ressort dépendant de la force de rappel à laquelle il est soumis et de sa température propre, chaque paire de ressorts antagoniste est avantageusement munie d'un capteur de température. Ces capteurs de température peuvent être constitués par de simples thermistances, dont la transmittance est linéaire sur la plage de températures définie par les températures de transition du matériau à mémoire de forme. Ces capteurs peuvent également être constitués par des thermocouples.

Ces différents capteurs précités sont reliés au bus de commande 9 qui est connecté à une unité de contrôle externe pour commander chaque maillon de la structure tubulaire. Sur réception des informations des différents capteurs, l'unité de commande asservira les ressorts appropriés pour déplacer dans l'espace la structure tubulaire.

L'endoscope, obtenu à partir de la structure tubulaire munie des différents capteurs, peut être déplacé de manière précise pour contourner les obstacles. Il permet de limiter les risques de perforations des parois des organes et d'accéder à des régions inaccessibles par des endoscopes de l'art antérieur.

Le bus de commande 9 doit permettre la lecture des capteurs de chaque maillon et la commande électrique des actionneurs. Afin de limiter la taille physique du bus de commande et ainsi d'augmenter le volume interne utile 6, un bus de commande basé sur le principe du multiplexage et l'utilisation éventuelle d'une logique ternaire permettent d'obtenir un bus de commande comprenant un nombre de fils déterminé et réduit au minimum, quel que soit le nombre de maillons. Chaque maillon porte une puce électronique, de l'ordre de 1 à 2 mm², comprenant des entrées pour les différents fils du bus de commande et des sorties pour le chauffage électrique des ressorts et la connexion aux différents capteurs. En fonction de l'électronique de commande de la puce utilisée, le bus de commande comprendra par exemple de 9 à 4 fils. Chaque maillon est scruté l'un après l'autre. Lorsqu'un maillon est validé, chacun de ses capteurs est lu, il est ensuite possible de changer l'état d'un de ses actionneurs en passant soit dans une phase active de chauffage ou une phase passive de refroidissement. Le maillon en cours de traitement est ensuite dévalidé et le maillon suivant est validé à son tour. Ce cycle se reproduit à l'infini d'une extrémité à l'autre de la structure tubulaire. Les données sont stockées en mémoire dans un tableau qu'un programme de commande de l'unité de contrôle vient consulter périodiquement. L'utilisation d'une logique ternaire permet de comprimer davantage le bus en compressant sur un fils du bus de commande 2bits d'informations. Un petit circuit analogique est alors prévu au niveau de la puce électronique de chaque maillon pour permettre de faire la discrimination et de décompresser l'information sur 2 fils. A titre d'exemple, en utilisant une puce appropriée, le bus de commande est limité à 4 fils, un fil de multiplexage permettant de passer d'un capteur à l'autre, un fil d'alimentation électrique pour le chauffage des ressorts, un fil de lecture des données fournies par les capteurs, et un fil de validation des maillons.

Dans une variante de réalisation, la structure tubulaire comprend des unités modulaires formées, non pas de maillons reliés par une liaison pivot, mais à partir d'un tube flexible continu obtenu par moulage, non métallique, avec des liaisons déformables. Le tube comprend des paires d'ouvertures ou espaces dans lesquelles sont logés les actionneurs, les ouvertures d'une paire étant disposées symétriquement et deux paires successives étant disposées à 90° l'une de l'autre. La portion de tube comprise entre deux paires successives constitue une unité modulaire et les deux portions de tube qui séparent les deux ouvertures d'une paire constituent des liaisons déformables reliant deux unités modulaires successives.

## Revendications

1. Structure tubulaire longitudinale active orientable (2), comprenant :
- des actionneurs en matériau à mémoire de forme, aptes à engendrer des mouvements de flexion de la structure tubulaire lorsqu'ils sont chauffés au-delà d'une température d'activation déterminée correspondant au passage du matériau d'une phase basse température à une phase haute température,
- des moyens de chauffage destinés à être connectés à des moyens de commande externes, lesdits moyens de commandes étant aptes à commander, de manière sélective, l'activation des actionneurs, et
- un empilement d'unités modulaires (3) articulées entre elles, deux unités modulaires (30a, 30b) successives étant aptes à pivoter selon au moins un axe de rotation transversal (R, R'), et étant espacées l'une de l'autre de façon à ménager un espace à dimension variable de part et d'autre dudit axe de rotation commun (R),
lesdits actionneurs étant constitués par des moyens de rappel élastiques (41, 42) en matériau à mémoire de forme dont la raideur varie en fonction de la température, montés entre lesdites unités modulaires, au moins un moyen de rappel étant monté entre deux unités modulaires successives sur un côté de leur axe de rotation commun, de sorte qu'à l'état inactif de l'actionneur, le moyen de rappel élastique rappelle les deux unités modulaires dans leur position d'alignement, et qu'à l'état actif de l'actionneur, un pivotement des deux unités modulaires par rapport à leur axe de rotation commun soit engendré en faisant varier la raideur du moyen de rappel élastique par chauffage pour atteindre la position d'orientation souhaitée, ledit au moins un moyen de rappel étant logé dans l'un des espaces (40) à dimension variable où il est sensiblement entièrement inséré, les unités modulaires comprenant des maillons rigides (30a, 30b) assemblés les uns aux autres de manière pivotante autour d'un axe de rotation (R) sensiblement perpendiculaire à l'axe de la structure tubulaire droite, les axes de rotation étant alternativement disposés sensiblement à 90° l'un de l'autre d'un maillon à l'autre, **caractérisée par le fait que** chaque maillon comprend une paroi (3a) de forme générale circulaire comprenant un bord supérieur (10) et un bord inférieur (11), ladite paroi, recourbée symétriquement dans un même sens longitudinal, comprenant deux parties recourbées (12a, 12b) diamétralement opposées, reliées par deux ponts (13a, 13b), lesdits espaces (40) à dimension variable de deux maillons successifs s'étendant du bord inférieur des parties recourbées d'un maillon au bord supérieur des ponts du maillon adjacent.

2. Structure tubulaire active selon la revendication 1, **caractérisée par le fait qu'**entre chaque paire d'unités modulaires (30a, 30b), sont montés deux moyens de rappel (41,42) de part et d'autre de leur axe de rotation commun (R), les positions d'alignement et d'orientation desdites unités modulaires correspondant à la position d'équilibre des deux moyens de rappel, lesdits deux moyens de rappel étant logés dans les deux espaces (40) à dimension variable.

3. Structure tubulaire active selon l'une des revendications 1 à 2, **caractérisée par le fait que** chaque unité modulaire (30a, 30b) présente une forme générale annulaire creuse, de sorte que leur empilement définisse un volume interne utile (6).

4. Structure tubulaire active selon l'une des revendications 2 à 3, **caractérisée par le fait que** chaque moyen de rappel est constitué par au moins un ressort (41, 42), assemblé par ses extrémités longitudinales à deux maillons consécutifs (30a, 30b), sollicité de préférence en traction à basse température, le pivotement relatif de deux maillons étant obtenu par augmentation de la raideur d'un des deux ressorts à effets antagonistes par chauffage de celui-ci au-delà de la température d'activation, la course retour en sens inverse étant obtenue après refroidissement du ressort activé en dessous d'une température de désactivation et par le rappel élastique de l'autre ressort antagoniste non activé.

5. Structure tubulaire active selon l'une des revendications 1 à 4, **caractérisée par le fait que** chaque maillon (30b) comprend deux languettes mâles (14a, 14b) diamétralement opposées et disposées longitudinalement et deux encoches femelles (17a, 17b) de forme sensiblement complémentaire diamétralement opposées, disposées sensiblement à 90° desdites languettes mâles, orientées en sens inverse de celles-ci et dans lesquelles sont emboîtées les languettes mâles du maillon adjacent (30a) pour former une liaison pivot entre les deux maillons définissant leur axe de rotation (R).

6. Structure tubulaire active selon la revendication 5, **caractérisée par le fait que** chaque languette mâle (14a, 14b) possède une portion périphérique circulaire (15a, 15b) s'étendant sur un arc de cercle supérieur à 180°, de sorte que l'emboîtement desdites portions d'un maillon (30b) dans les encoches femelles complémentaires (17a, 17b) du maillon (30a) adjacent permet de retenir longitudinalement les deux maillons.

7. Structure tubulaire active selon la revendication 5 ou 6, **caractérisée par le fait que** chaque maillon (30b) comporte une bague (20) comprenant deux parties (25a, 25b) diamétralement opposées venant transversalement en vis-à-vis des encoches femelles (17a, 17b) dudit maillon afin d'empêcher un déboîtement transversal des languettes mâles du maillon adjacent (30a).

8. Structure tubulaire active selon l'une des revendications 4 à 7, **caractérisée par le fait que** les extrémités longitudinales des ressorts (41, 42) sont assemblées aux maillons par l'intermédiaire de plots en céramique (44, 43), lesdits plots portant des fils et connexions électriques (54, 55), constituant les moyens de chauffage, qui permettent le chauffage desdits ressorts.

9. Structure tubulaire active selon la revendication 8, **caractérisée par le fait que** chaque moyen de rappel comprend deux ressorts (41, 42), disposés symétriquement par rapport à un plan perpendiculaire à l'axe de rotation commun (R) des deux maillons (30a, 30b) auxquels ils sont associés, et assemblés auxdits maillons par les mêmes plots en céramique (44, 43).

10. Structure tubulaire active selon l'une des revendications 4 à 9, **caractérisée par le fait que** chaque ressort (41, 42) présente une forme générale repliée en accordéon et est constitué d'un ensemble de bandes flexibles (45, 46a-d, 47) superposées, reliées par des parties de liaison rigides (49).

11. Structure tubulaire active selon l'une des revendications précédentes, **caractérisée par le fait qu'**elle comprend au niveau des actionneurs des capteurs d'orientation destinés à être reliés aux moyens de commande pour le contrôle de l'orientation relative des unités modulaires, tels que des capteurs de flexion (56) permettant de déterminer la position angulaire relative entre deux unités modulaires consécutives et/ou des capteurs de contact (58) permettant de mesurer localement la force d'interaction entre la surface extérieure de la structure tubulaire et le milieu extérieur, et/ou des capteurs de température sur chaque actionneur. 3

12. Structure tubulaire active selon les revendications 3 et 11 prises en combinaison, **caractérisée par le fait qu'**elle comprend un bus de communication (9) par multiplexage disposé dans le volume interne (6) et destiné à assurer la liaison entre les moyens de commande externes, les moyens de chauffage et les capteurs pour commander électriquement, de manière sélective, l'activation des actionneurs en fonction des informations fournies par les capteurs et des ordres de commande fournis par l'utilisateur.

13. Endoscope (1) comprenant un corps tubulaire longitudinal muni à son extrémité distale (1a) d'une tête (5) dans laquelle est disposé un système de vision destiné à être relié par des canaux de communication (7) à une unité de contrôle et de commande externe et/ou un ou plusieurs micro-outils destinés à être reliés par des câbles électriques (8) à ladite unité de commande et de contrôle externe, lesdits canaux et/ou câbles étant disposés dans le corps tubulaire, **caractérisé par le fait qu'**au moins une partie du corps de l'endoscope est formée à partir de la structure tubulaire active orientable (2) selon l'une des revendications 1 à 12, les unités modulaires de la structure étant recouvertes par au moins une gaine externe flexible (4).

## Claims

1. Active steerable longitudinal tubular structure (2), comprising:
- actuators made from shape-memory material and able to generate flexion movements of the tubular structure when they are heated beyond a defined activation temperature corresponding to the passage of the material from a low-temperature phase to a high-temperature phase,
- heating means designed to be connected to external control means, said control means being able to selectively control the activation of the actuators, and
- a stack of mutually articulated modular units (3), two successive modular units (30a, 30b) being able to pivot on at least one transverse axe of rotation (R, R') and being spaced apart from one another in such a way as to form a space of variable size on each side of the said axe of rotation (R),
said actuators being formed by elastic return means (41, 42), which are made of shape-memory material, the stiffness of which varies as a function of the temperature, and which are mounted between said modular units, at least one return means being mounted between two successive modular units on one side of their common axis of rotation, in such a way that, when the actuator is in the inactive state, the elastic return means returns the two modular units to their position of alignment, and, when the actuator is in the active state, a pivoting of the two modular units with respect to their common axis of rotation is generated by varying the stiffness of the elastic return means by heating in order to obtain the desired orientation position, said at least one return means being housed in one of the spaces (40) of variable size, where it is more or less completely inserted, the modular units comprising rigid links (30a, 30b) joined to one another so as to pivot about one axe of rotation (R) substantially perpendicular to the axis of the straight tubular structure, the axes of rotation being alternatively arranged substantially at 90° to one another from one link to another, each link comprising a wall (3a) of general circular shape having an upper edge (10) and a lower edge (11), said wall, curved symmetrically in the same longitudinal direction, having two diametrically opposite curved parts (12a, 12b) that are connected by two bridges (13a, 13b), **characterized in that** said spaces (40) of variable size of two successive links extend from the lower edge of the curved parts of one link to the upper edge of the bridges of the adjacent link.

2. Active tubular structure according to Claim 1, **characterized in that**, between each pair of modular units (30a, 30b), two return means (41, 42) are mounted either side of their common axis of rotation (R), the positions of alignment and of orientation of said modular units corresponding to the position of equilibrium of the two return means, said two return means being housed in the two spaces (40) of variable size.

3. Active tubular structure according to one of Claims 1 and 2, **characterized in that** each modular unit (30a, 30b) has what is generally a hollow annular shape, such that their stacking defines a useful internal volume (6).

4. Active tubular structure according to one of Claims 2 and 3, **characterized in that** each return means is formed by at least one spring (41, 42) joined via its longitudinal ends to two consecutive links (30a, 30b) and preferably under tensile stress at low temperature, the relative pivoting of two links being obtained by increasing the stiffness of one of the two antagonistic springs by heating it beyond the activation temperature, the return movement in the opposite direction being obtained after cooling the activated spring to below a deactivation temperature and by the elastic return of the other antagonistic spring not activated.

5. Active tubular structure according to one of Claims 1 to 4, **characterized in that** each link (30b) comprises two male tongues (14a, 14b) which are located diametrically opposite one another and arranged longitudinally, and two female notches (17a, 17b) of substantially complementary shape which are located diametrically opposite one another and arranged substantially at 90° to said male tongues and are oriented in the opposite direction to the latter, and in which the male tongues of the adjacent link (30a) are engaged in order to form a pivot connection between the two links defining their axis of rotation (R).

6. Active tubular structure according to Claim 5, **characterized in that** each male tongue (14a, 14b) has a circular peripheral portion (15a, 15b) extending on an arc of a circle greater than 180°, such that the engagement of said portions of a link (30b) in the complementary female notches (17a, 17b) of the adjacent link (30a) allows the two links to be retained longitudinally.

7. Active tubular structure according to Claim 5 or 6, **characterized in that** each link (30b) comprises a ring (20) with two diametrically opposite parts (25a, 25b) which come into position transversely opposite the female notches (17a, 17b) of said link in order to avoid transverse disengagement of the male tongues of the adjacent link (30a).

8. Active tubular structure according to one of Claims 4 to 7, **characterized in that** the longitudinal ends of the springs (41, 42) are joined to the links by way of ceramic pins (44, 43), said pins carrying wires and electrical connections (54, 55) forming the heating means that permit heating of said springs.

9. Active tubular structure according to Claim 8, **characterized in that** each return means comprises two springs (41, 42), which are arranged symmetrically with respect to a plane perpendicular to the common axis of rotation (R) of the two links (30a, 30b) to which they are joined, and which are connected to said links by the same ceramic pins (44, 43).

10. Active tubular structure according to one of Claims 4 to 9, **characterized in that** each spring (41, 42) has what is generally a folded accordion shape and is formed by a set of superposed flexible bands (45, 46a-d, 47) that are connected by rigid connecting parts (49).

11. Active tubular structure according to one of the preceding claims, **characterized in that** it comprises, in the area of the actuators, orientation sensors designed to be connected to the control means for controlling the relative orientation of the modular units, such as flexion sensors (56) for determining the relative angular position between two consecutive modular units and/or contact sensors (58) for locally measuring the force of interaction between the outer surface of the tubular structure and the external environment, and/or temperature sensors on each actuator.

12. Active tubular structure according to Claims 3 and 11 in combination, **characterized in that** it comprises a multiplex communication bus (9) arranged in the inner volume (6) and designed to establish the connection between the external control means, the heating means and the sensors, in order to permit selective electrical control of the activation of the actuators as a function of the information supplied by the sensors and the commands entered by the user.

13. Endoscope (1) comprising a longitudinal tubular body equipped at its distal end (1a) with a head (5) in which a viewing system is arranged that is designed to be connected by communication channels (7) to an external control and command unit and/or one or more micro-tools designed to be connected by electrical cables (8) to said external control and command unit, said channels and/or cables being arranged in the tubular body, **characterized in that** at least part of the body of the endoscope is formed from the active steerable tubular structure (2) according to one of Claims 1 to 12, the modular units of the structure being covered by at least one flexible outer sheath (4).

## Patentansprüche

1. Schwenkbare, aktive, longitudinal röhrenförmige Struktur (2), umfassend:
- Aktuatoren aus Formgedächtnismaterial, die befähigt sind, bei Erwärmung oberhalb einer bestimmten Aktivierungstemperatur, die dem Übergang des Materials von einer Niedertemperaturphase zu einer Hochtemperaturphase entspricht, Biegungsbewegungen der röhrenförmigen Struktur hervorzurufen,
- Heizmittel, die zum Anschluss an externe Steuerungsvorrichtungen bestimmt sind, wobei die Steuerungsvorrichtungen befähigt sind, die Aktivierung der Aktuatoren selektiv zu steuern, und
- einen Stapel modularer Einheiten (3), die untereinander durch Gelenke verbunden sind, wobei zwei aufeinanderfolgende modulare Einheiten (30a, 30b) um zumindest eine transversale Rotationsachse (R, R') drehbar und so voneinander beabstandet angeordnet sind, dass auf beiden Seiten der Rotationsachse (R) ein Zwischenraum von variabler Größe entsteht,
wobei die Aktuatoren aus elastischen Rückstellmitteln (41, 42) aus Formgedächtnismaterial, dessen Steifigkeit sich in Abhängigkeit der Temperatur ändert, bestehen, die zwischen den modularen Einheiten angebracht sind, wobei wenigstens ein Rückstellmittel zwischen zwei aufeinanderfolgenden modulare Einheiten auf einer Seite von deren gemeinsamer Rotationsachse derart angebracht ist, dass im inaktiven Zustand des Aktuators das elastische Rückstellmittel die beiden modularen Einheiten in ihre fluchtende Stellung zurückstellt und dass im aktiven Zustand des Aktuators eine Drehung der beiden modularen Einheiten bezogen auf ihre gemeinsame Rotationsachse durch Änderung der Steifigkeit des Rückstellmittels durch Erwärmen hervorgerufen wird, um die gewünschte Schwenkstellung zu erreichen, wobei das wenigstens eine Rückstellmittel in einem der Zwischenräume variabler Größe (40) untergebracht ist, worin dieses im Wesentlichen vollständig eingefügt ist,
wobei die modularen Einheiten starre Glieder (30a, 30b) umfassen, die drehbar um die Rotationsachse (R) im Wesentlichen senkrecht zur Achse der geraden röhrenförmigen Struktur aneinander montiert sind, wobei die Rotationsachsen von einem Glied zum anderen abwechselnd in einem Winkel von im Wesentlichen 90° zueinander angeordnet sind,
wobei jedes Glied eine Wandung (3a) von kreisförmiger Allgemeinform mit einer oberen Kante (10) und einer unteren Kante (11), umfasst,
wobei die symmetrisch in gleicher Längsrichtung gebogene Wandung zwei diametral entgegengesetzte gebogene Teile (12a, 12b) umfasst, die durch zwei Brücken (13a, 13b) verbunden sind, **dadurch gekennzeichnet, dass** die Zwischenräume variabler Größe (40) zweier aufeinanderfolgender Glieder sich von der unteren Kante der gebogenen Teile eines Gliedes zur oberen Kante der Brücken des benachbarten Gliedes erstrecken.

2. Aktive, röhrenförmige Struktur gemäß Anspruch 1 **dadurch gekennzeichnet, dass** zwischen jedem Paar modularer Einheiten (30a, 30b) zwei Rückstellmittel (41, 42) auf beiden Seiten von deren gemeinsamer Rotationsachse (R) angebracht sind, wobei die fluchtende Stellung und die Schwenkstellung der modulareren Einheiten der Gleichgewichtsposition der beiden Rückstellmittel entsprechen und die beiden Rückstellmittel in den beiden Zwischenräumen variabler Größe (40) angeordnet sind.

3. Aktive, röhrenförmige Struktur gemäß einem der Ansprüche 1 bis 2 **dadurch gekennzeichnet, dass** jede modulare Einheit (30a, 30b) eine ringförmige, hohle Allgemeinform aufweist, so dass deren Stapel einen nutzbaren Innenraum (6) definiert.

4. Aktive, röhrenförmige Struktur gemäß einem der Ansprüche 2 bis 3 **dadurch gekennzeichnet, dass** jedes Rückstellmittel aus wenigstens einer Feder (41, 42) aufgebaut ist, die durch ihre längsständigen Enden mit zwei aufeinanderfolgenden Gliedern (30a, 30b) verbunden ist und bei niedrigen Temperaturen bevorzugt durch Zug beansprucht wird, wobei die Relativdrehung zweier Glieder durch Erhöhung der Steifigkeit einer der beiden Federn mit entgegengesetzter Wirkung durch Erwärmen dieser Feder oberhalb der Aktivierungstemperatur erhalten wird, und der Rückweg in entgegengesetzter Richtung nach Abkühlen der aktivierten Feder unterhalb einer Deaktivierungstemperatur und durch das elastische Rückstellen der anderen, antagonistischen, nicht aktivierten Feder erhalten wird.

5. Aktive, röhrenförmige Struktur gemäß einem der Ansprüche 2 bis 4 **dadurch gekennzeichnet, dass** jedes Glied (30b) zwei diametral entgegengesetzte und in Längsrichtung angeordnete Laschen (14a, 14b) und zwei diametral entgegengesetzte Aussparungen (17a, 17b) von im Wesentlichen komplementärer Form umfasst, die im Wesentlichen im Winkel von 90° zu den Laschen angeordnet und in entgegengesetzter Richtung zu diesen ausgerichtet sind und in denen die Laschen des benachbarten Gliedes (30a) eingefügt sind, wodurch eine drehbare Verbindung zwischen den beiden Gliedern, die deren Rotationsachse (R) definiert, ausgebildet wird.

6. Aktive, röhrenförmige Struktur gemäß Anspruch 5 **dadurch gekennzeichnet, dass** jede Lasche (14a, 14b) einen kreisförmigen peripheren Teil (15a, 15b) aufweist, der sich über einen Kreisbogen von mehr als 180° erstreckt, wodurch die Einfügung der Teile eines Gliedes (30b) in den komplementären Aussparungen (17a, 17b) des benachbarten Gliedes (30a) erlaubt, die beiden Glieder in Längsrichtung festzuhalten.

7. Aktive, röhrenförmige Struktur gemäß Anspruch 5 oder 6 **dadurch gekennzeichnet, dass** jedes Glied (30b) einen Ring (20) trägt, der zwei diametral entgegengesetzte Teile (25a, 25b) umfasst, die in Querrichtung gegenüber den Aussparungen (17a, 17b) des Glieds angeordnet werden, um ein Lösen der Laschen des benachbarten Gliedes (30a) in Querrichtung zu verhindern.

8. Aktive, röhrenförmige Struktur gemäß einem der Ansprüche 4 bis 7 **dadurch gekennzeichnet, dass** die längsständigen Enden der Federn (41, 42) mit den Gliedern über Keramikkontakte (44, 43) verbunden sind, wobei die Kontakte Elektrokabel und -verbindungen (54, 55) aufweisen, die die Heizmittel bilden, die das Erwärmen der Federn ermöglichen.

9. Aktive, röhrenförmige Struktur gemäß Anspruch 8 **dadurch gekennzeichnet, dass** jedes Rückstellmittel zwei Federn (41, 42) umfasst, die bezogen auf eine, zur gemeinsamen Rotationsachse (R) der beiden Glieder (30a, 30b), mit denen diese Federn verbunden sind, senkrechte Ebene symmetrisch angeordnet und mit den Gliedern durch die gleichen Keramikkontakte (44, 43) verbunden sind.

10. Aktive, röhrenförmige Struktur gemäß einem der Ansprüche 4 bis 9 **dadurch gekennzeichnet, dass** jede Feder (41, 42) eine akkordeonartig gefaltete Allgemeinform aufweist und aus einer Gesamtheit übereinanderliegender, flexibler Bänder (45, 46a-d, 47) aufgebaut ist, die durch starre Bindungsteile (49) verbunden sind.

11. Aktive, röhrenförmige Struktur gemäß einem der vorhergehenden Ansprüche **dadurch gekennzeichnet, dass** diese auf Höhe der Aktuatoren Ausrichtungssensoren umfasst, die vorgesehen sind, an die Steuerungsvorrichtungen angeschlossen zu werden, um die relative Ausrichtung der modularen Einheiten zu steuern, wie Biegungssensoren (56), die es erlauben, die relative Winkelposition zwischen zwei aufeinanderfolgenden modularen Einheiten zu bestimmen, und/oder Kontaktsensoren (58), die es erlauben, lokal die Wechselwirkungs-Kraft zwischen äußerer Oberfläche der röhrenförmigen Struktur und der äußeren Umgebung zu messen, und/oder Temperatursensoren auf jedem Aktuator.

12. Aktive, röhrenförmige Struktur gemäß den kombinierten Ansprüchen 3 und 11 **dadurch gekennzeichnet, dass** diese einen Multiplex-Kommunikationsbus (9) umfasst, der im Innenraum (6) angeordnet und dazu bestimmt ist, die Verbindung zwischen den externen Steuerungseinheiten, den Heizmitteln und den Sensoren sicherzustellen, um selektiv die Aktivierung der Aktuatoren in Abhängigkeit von den durch die Sensoren bereitgestellten Informationen und den durch den Benutzer bereitgestellten Steuerungsbefehle elektrisch zu steuern.

13. Endoskop (I) umfassend einen longitudinal röhrenförmigen Körper, der an seinem distalen Ende (1a) einen Kopf (5) aufweist, in dem eine Sehvorrichtung, die vorgesehen ist, durch Kommunikationskanäle (7) mit einer externen Kontroll- und Steuereinheit verbunden zu werden, und/oder ein oder mehrere Mikrowerkzeuge, die vorgesehen sind, durch Elektrokabel (8) mit der externen Kontroll- und Steuereinheit verbunden zu werden, angeordnet sind, wobei die Kanäle und/oder Kabel in dem röhrenförmigen Körper angeordnet sind, **dadurch gekennzeichnet, dass** wenigstens ein Teil des Körpers des Endoskops durch die steuerbare, aktive, röhrenförmige Struktur (2) gemäß einem der Ansprüche 1 bis 12 gebildet wird, wobei die modularen Einheiten der Struktur durch wenigstens eine äußere flexible Hülle (4) bedeckt werden.
